# EUROPÄISCHE PATENTSCHRIFT

(11) **EP 4 124 356 B1**
(45) Veröffentlichungstag und Bekanntmachung des Hinweises auf die Patenterteilung: **11.03.2026**
(21) Anmeldenummer: 21188130.5
(22) Anmeldetag: 28.07.2021
(51) Int. Cl.: A61M 39/24, A61B 17/86

(54) **VORRICHTUNG UND VERFAHREN ZUR APPLIKATION EINES PHARMAZEUTISCHEN FLUIDS**
DEVICE AND METHOD FOR APPLICATION OF A PHARMACEUTICAL FLUID
DISPOSITIF ET PROCÉDÉ D'APPLICATION D'UN FLUIDE PHARMACEUTIQUE

(43) Veröffentlichungstag der Anmeldung: 01.02.2023
(73) Patentinhaber: Heraeus Medical GmbH, 61273 Wehrheim (DE)
(72) Erfinder: Dr. VOGT, Sebastian, 63450 Hanau (DE); Dr. KLUGE, Thomas, 63450 Hanau (DE)
(74) Vertreter: Heraeus IP

(56) Entgegenhaltungen:
- US-A1- 2015 080 813
- US-A1- 2019 167 326

## Beschreibung

Die Erfindung betrifft eine Vorrichtung zur Applikation eines pharmazeutischen Fluids umfassend einen hohlzylinderförmigen Körper, der einen Kanal umgibt, der sich von einem proximalen Körperende zu einem distalen Körperende des Körpers durch den Körper erstreckt, wobei eine Außenfläche des Körpers zumindest teilweise ein Außengewinde aufweist, und ein am proximalen Körperende angeordnetes Verbindungselement, über das ein proximales Kanalende des Kanals fluidleitend mit einem Reservoir für das pharmazeutische Fluid reversibel verbindbar ist.

Gegenstand der Erfindung ist insbesondere eine medizinische Vorrichtung zur temporären, lokalen Applikation von pharmazeutischen Fluiden oder anderen medizinischen Fluiden über einen Zeitraum von Stunden bis zu mehreren Monaten. Die erfindungsgemäße Vorrichtung ist vor allem für die Behandlung entzündlicher Erkrankungen von Gelenken, wie die aktivierte Arthrose und die rheumatische Arthritis, bestimmt.

### Hintergrund der Erfindung

Entzündliche Erkrankungen der Gelenke, wie die aktivierte Arthrose und die rheumatische Arthritis, sind weit verbreitet. Diese Erkrankungen sind häufig mit Gelenkschmerzen verbunden und können durch einen fortschreitenden Entzündungsprozess eine zunehmende Gelenkzerstörung bewirken, welche zu einer Beeinträchtigung der Gelenkfunktion oder gar zu einem vollständigen Verlust der Gelenkfunktion führen können.

Neben einer systemischen pharmakologischen Therapieoption, welche mit Nebenwirkungen für den Patienten behaftet sein kann und welche aufgrund einer vergleichsweise geringen Wirkstoffkonzentration an der Entzündungsstelle weniger wirksam ist, gibt es die Möglichkeit, durch intraartikuläre Injektion von pharmazeutischen Fluiden, insbesondere von entzündungshemmenden Wirkstoffen, den Entzündungsprozess zu beeinflussen. Als Wirkstoffe kommen hierbei beispielsweise Dexamethasonphosphat, Cyclosporin, Sulfasalazin und Methotrexat in Betracht.

Kritisch ist bei der intraartikulären Injektion zu sehen, dass eine nicht unerhebliche Infektionsgefahr für den intraartikulären Raum besteht. Weiterhin sind die Gelenkkapseln des Menschen mit Nozizeptoren ausgestattet, wodurch die Gelenkkapsel sehr schmerzempfindlich sind.

Knochenschrauben mit einem zentralen Kanal im Schraubenkörper und damit verbundenen Austrittsöffnungen sind unter der Bezeichnung kanülierte und fenestrierte Schrauben bekannt. Bisher werden diese Knochenschrauben im Wesentlichen im Spine-Bereich als sogenannte kanülierte und fenestrierte Pedikelschrauben eingesetzt. Dabei wird durch den Kanal der Schrauben Knochenzementteig in den meist osteoporotischen Wirbelkörper eingespritzt, wodurch sich überwiegend ein zur Längsachse der Pedikelschraube koaxialer Zementmantel ausbildet. Der Knochenzement härtet anschließend aus und bildet ein Widerlager für die Pedikelschrauben. Eine Vielzahl von kanülierten und fenestrierten Knochenschrauben wurden vorgeschlagen. Exemplarisch sind dafür die Patenschriften und Offenlegungsschriften DE 35 08 759 A1, DE 1994 9285 C2, DE 10 2011 112 890 B4, EP 1 210 019 B1, EP 2 887 899 B1 genannt.

Daneben können kanülierte und fenestrierte Knochenschrauben auch zur lokalen Applikation pharmazeutischer Fluide eingesetzt werden, wie dies exemplarisch in den Patentschriften und Offenlegungsschriften US 9 616 205 B2, US 10 357 298 B2 und US 2018/0014867 A1 beschrieben wurde. US 2019/167326 offenbart eine Knochenschraube, die ein Rückschlagventil umfasst.

Nachteilig an den bisher beschriebenen kanülierten und fenestrierten Knochenschrauben gestaltet sich ein Einwachsen von Gewebe, insbesondere von Bindegewebe, in die Austragsöffnungen der Knochenschrauben, welches ein weiteres Applizieren pharmazeutischer Fluide mittels der derart fluidleitend verschlossenen Knochenschraube erschwert oder gar vollständig unterbindet.

Wünschenswert ist daher eine Vorrichtung, die gegen ein Einwachsen von Gewebe, insbesondere von Bindegewebe, geschützt ist, so dass eine Applikation eines pharmazeutischen Fluids über einen längeren Zeitraum, beispielsweise über mehrere Wochen oder gar Monate, möglich ist. Weiterhin sollte die Vorrichtung eine lokale Applikation beliebiger pharmazeutischer Wirkstoffe in Form pharmazeutischer Fluide zulassen, wobei es jederzeit möglich ist, die Zusammensetzung und/oder die Konzentration an Wirkstoffen in dem pharmazeutischen Fluid auszutauschen. Außerdem ist es wünschenswert, dass die Wirkstoffkonzentration im pharmazeutischen Fluid, die unmittelbar am Implantationsort der Vorrichtung erreicht wird, direkt von außen eingestellt werden kann.

### Aufgaben

Eine Aufgabe der vorliegenden Erfindung ist es, einen oder mehrere der sich aus dem Stand der Technik ergebenen Nachteile zumindest teilweise zu überwinden.

Insbesondere soll eine Vorrichtung zur lokalen Applikation pharmazeutischer Fluide, wie zum Beispiel von antiphlogistische, cytostatische und glucocorticoidische Lösungen, bereitgestellt werden, mit der eine lokale und temporäre Abgabe des pharmazeutischen Fluids in Gelenkkapseln eines Patienten, insbesondere Gelenkkapseln größerer Gelenke wie beispielsweise dem Kniegelenk, ermöglicht wird.

Die Vorrichtung soll so beschaffen sein, dass von außerhalb des Patientenkörpers dem Patienten bei Bedarf pharmazeutische Fluide beliebiger und wechselnder Zusammensetzung mehrfach in eine Gelenkkapsel eingebracht werden können, ohne dass dabei die Gelenkkapsel durchstochen werden muss. Die Vorrichtung soll auch über längere Zeiträume, beispielsweise über einen Zeitraum von mehreren Wochen oder mehreren Monaten im Patienten zur wiederholten Abgabe des pharmazeutischen Fluids geeignet sein, ohne dass die Vorrichtung hierfür entfernt werden muss und ohne dass es zu einem Verstopfen der Vorrichtung, insbesondere durch Ablagerung körpereigenem Gewebes, insbesondere körpereigener Proteine, kommt. Weiterhin soll Synovialflüssigkeit nicht durch die Vorrichtung aus der Gelenkkapsel austreten. Die Vorrichtung soll durch ihren Aufbau verhindern, dass Mikroorganismen in den intraartikulären Raum eindringen können. Die Vorrichtung soll kostengünstig zu fertigen sein und möglichst ein hygienisches, nur einmal verwendbares Wegwerfprodukt sein. Die Fluidabgabe aus der Vorrichtung soll von außen kontrolliert werden können.

### Bevorzugte Ausführungsformen der Erfindung

Ein Beitrag zur mindestens teilweisen Erfüllung mindestens einer der zuvor genannten Aufgaben wird durch die Merkmale der unabhängigen Ansprüche geleistet. Die abhängigen Ansprüche stellen bevorzugte Ausführungsformen bereit, die zur mindestens teilweisen Erfüllung mindestens einer der Aufgaben beitragen.

Eine erste Ausführungsform der Erfindung ist eine Vorrichtung zur Applikation eines pharmazeutischen Fluids umfassend einen hohlzylinderförmigen Körper, der einen Kanal umgibt, der sich von einem proximalen Körperende zu einem distalen Körperende des Körpers durch den Körper erstreckt, wobei eine Außenfläche des Körpers zumindest teilweise ein Außengewinde aufweist, ein am proximalen Körperende angeordnetes Verbindungselement, über das ein proximales Kanalende des Kanals reversibel fluidleitend mit einem Reservoir für das pharmazeutische Fluid verbindbar ist, gekennzeichnet durch ein im Kanal angeordnetes Rückschlagventil, welches in Richtung des proximalen Kanalendes fluidundurchlässig ausgestaltet ist, und ein im Kanal distal zum Rückschlagventil angeordnetes Dichtungselement, welches ein distales Kanalende des Kanals fluidleitend verschließt und welches einen Spalt aufweist, welcher bei einer Druckbeaufschlagung auf das pharmazeutische Fluid aus Richtung des proximalen Kanalendes reversibel fluidleitend öffenbar ist, so dass das pharmazeutische Fluid aus dem distalen Kanalende applizierbar ist.

In einer Ausführungsform der Vorrichtung öffnet sich der Spalt bei einer Druckbeaufschlagung von größer als 5 N/cm², bevorzugt größer als 6 N/cm², weiter bevorzugt größer als 7 N/cm², reversibel fluidleitend, so dass das distale Kanalende fluidleitend geöffnet ist. Diese Ausführungsform ist eine zweite Ausführungsform der Erfindung, welche vorzugsweise von der ersten Ausführungsform der Erfindung abhängt.

In der Vorrichtung ist das Rückschlagventil ein Kugelrückschlagventil umfassend eine Kugel und ein Rückstellelement.

In einer Ausführungsform der Vorrichtung sind das Rückstellelement und das Dichtungselement einteilig ausgestaltet. Diese Ausführungsform ist eine vierte Ausführungsform der Erfindung, welche vorzugsweise von der dritten Ausführungsform der Erfindung abhängt.

In einer Ausführungsform der Vorrichtung ist im Kanal ein Filterelement angeordnet. Diese Ausführungsform ist eine fünfte Ausführungsform der Erfindung, welche vorzugsweise von einer der vorhergehenden Ausführungsformen der Erfindung abhängt.

In einer Ausführungsform der Vorrichtung ist das Filterelement im Kanal proximal zum Rückschlagventil, also näher am proximalen Kanalende als das Rückschlagventil, angeordnet. Diese Ausführungsform ist eine sechste Ausführungsform der Erfindung, welche vorzugsweise von der fünften Ausführungsform der Erfindung abhängt.

In einer Ausführungsform der Vorrichtung ist das Filterelement eine mikroporöse Filterplatte. Diese Ausführungsform ist eine siebte Ausführungsform der Erfindung, welche vorzugsweise von der fünften oder der sechsten Ausführungsform der Erfindung abhängt.

In einer Ausführungsform der Vorrichtung weist die mikroporöse Filterplatte Poren mit einem durchschnittlichen Porendurchmesser von kleiner 40 µm, bevorzugt von kleiner 5 µm, weiter bevorzugt von kleiner 1 µm, auf. Diese Ausführungsform ist eine achte Ausführungsform der Erfindung, welche vorzugsweise von der siebten Ausführungsform der Erfindung abhängt.

In einer Ausführungsform der Vorrichtung ist im Kanal distal zum Dichtungselement, also näher am distalen Kanalende als das Dichtungselement, eine hohlzylinderförmige Hülse angeordnet, um ein Austragen des Dichtungselements, und damit bevorzugt auch aller weiterenn Strukturelemente im Kanal proximal zum Dichtungselement, aus dem distalen Kanalende beim Applizieren des pharmazeutischen Fluids zu unterbinden. Diese Ausführungsform ist eine neunte Ausführungsform der Erfindung, welche vorzugsweise von einer der vorhergehenden Ausführungsformen der Erfindung abhängt.

In einer Ausführungsform der Vorrichtung ist das proximale Kanalende mehrkantförmig, insbesondere sechskantförmig, ausgebildet und das Verbindungselement mit einem dazu passenden mehrkantförmigen, insbesondere sechskantförmigen, Verbindungselementabschnitt ausgestattet, um das Verbindungselement mit dem proximalen Kanalende reversibel fluidleitend durch Einstecken des Verbindungselementabschnitts in das proximale Kanalende zu verbinden.

Diese Ausführungsform ist eine zehnte Ausführungsform der Erfindung, welche vorzugsweise von einer der vorhergehenden Ausführungsformen der Erfindung abhängt.

In einer Ausführungsform der Vorrichtung weist das mehrkantförmige, insbesondere sechskantförmige, proximale Kanalende eine größere axiale Erstreckung auf als der mehrkantförmige, insbesondere sechskantförmige Verbindungselementabschnitt, so dass nach einem Entfernen, insbesondere durch Abschneiden oder Absägen, eines Teils, insbesondere eines proximalen Teils des proximalen Kanalendes, das Verbindungselement mit einem an der Vorrichtung verbleibenden Teil des proximalen Kanalendes reversibel fluidleitend durch Einstecken des Verbindungselementabschnitts in das verbleibende proximale Kanalende verbindbar ist. Diese Ausführungsform ist eine elfte Ausführungsform der Erfindung, welche vorzugsweise von der zehnten Ausführungsform der Erfindung abhängt.

In einer Ausführungsform der Vorrichtung umfasst der Körper ein Metall, ein Polymer oder ein Metall und ein Polymer, insbesondere besteht der Körper aus einem Metall, einem Polymer oder einem Metall und einem Polymer. Diese Ausführungsform ist eine zwölfte Ausführungsform der Erfindung, welche vorzugsweise von einer der vorhergehenden Ausführungsformen der Erfindung abhängt.

Ein Gegenstand der Offenbarung ist ein Verfahren zur Applikation eines pharmazeutischen Fluid mittels einer Vorrichtung nach einer der ersten bis zwölften Ausführungsform der Erfindung aufweisend die folgenden Schritte:
a. Implantieren der Vorrichtung in;
b. Fluidleitendes Verbinden der Vorrichtung mit einem Reservoir für das pharmazeutischen Fluids;
c. Aufbau eines Förderdrucks auf das pharmazeutische Fluid aus Richtung des proximalen Kanalendes von größer 5 N/cm²;
d. Fluidleitendes Öffnen des Spalts im Dichtungselements durch Einwirkung des Förderdrucks;
e. Ausbringen des pharmazeutischen Fluids aus dem fluidleitend geöffneten Spalt;
f. Fluidleitendes Verschließen des Spalts durch Reduktion des Förderdrucks auf 5 N/cm² oder weniger.

### Allgemeines

In der vorliegenden Beschreibung beinhalten Bereichsangaben auch die als Grenzen genannten Werte. Eine Angabe der Art "im Bereich von X bis Y" in Bezug auf eine Größe A bedeutet folglich, dass A die Werte X, Y und Werte zwischen X und Y annehmen kann. Einseitig begrenzte Bereiche der Art "bis zu Y" für eine Größe A bedeuten entsprechend als Wert Y und kleiner als Y.

Einige der beschriebenen Merkmale sind mit dem Begriff "im Wesentlichen" verknüpft. Der Begriff "im Wesentlichen" ist so zu verstehen, dass unter realen Bedingungen und Fertigungstechniken eine mathematisch exakte Auslegung von Begrifflichkeiten wie "Überlagerung", "senkrecht", "Durchmesser" oder "Parallelität" nie exakt, sondern nur innerhalb gewisser fertigungstechnischer Fehlertoleranzen gegeben sein kann. Beispielsweise schließen "im Wesentlichen senkrechte Achsen" einen Winkel von 85 Grad bis 95 Grad zueinander ein und "im Wesentlichen gleiche Volumen" umfassen eine Abweichung von bis zu 5 Volumen-%. Eine "im Wesentlichen aus Kunststoff bestehende Vorrichtung" umfasst beispielsweise einen Kunststoffanteil von ≥95 bis ≤100 Gewichts-%. Eine "im Wesentlichen vollständige Befüllung eines Volumens B" umfasst beispielsweise eine Befüllung von ≥95 bis ≤100 Volumen-% des Gesamtvolumens von B.

### Ausführliche Beschreibung

Ein erster Gegenstand der Erfindung betrifft eine Vorrichtung zur Applikation eines pharmazeutischen Fluids, insbesondere zur Applikation eines pharmazeutischen Fluids in eine Gelenkkapsel, umfassend einen hohlzylinderförmigen Körper, der einen Kanal umgibt, der sich von einem proximalen Körperende zu einem distalen Körperende des Körpers durch den Körper erstreckt,
wobei eine Außenfläche des Körpers zumindest teilweise ein Außengewinde aufweist,
ein am proximalen Körperende angeordnetes Verbindungselement, über das ein proximales Kanalende des Kanals reversibel fluidleitend mit einem Reservoir für das pharmazeutische Fluid verbindbar ist,
gekennzeichnet durch
ein im Kanal angeordnetes Rückschlagventil, welches in Richtung des proximalen Kanalendes fluidundurchlässig ausgestaltet ist, und ein im Kanal distal zum Rückschlagventil angeordnetes Dichtungselement, welches ein distales Kanalende des Kanals fluidleitend verschließt und in dem bei einer Druckbeaufschlagung auf das pharmazeutische Fluid aus Richtung des proximalen Kanalendes reversibel ein Spalt fluidleitend öffenbar ist, so dass das pharmazeutische Fluid aus dem distalen Kanalende applizierbar ist.

Die Vorrichtung weist im Kanal ein Rückschlagventil auf. Ein Rückschlagventil ist ein Bauteil, welches ein Fördern des pharmazeutischen Fluids in eine Förderrichtung erlaubt, während ein Fördern in die entgegengesetzte Förderrichtung unterbunden wird. Das erfindungsgemäße Rückschlagventil erlaubt ein Fördern des pharmazeutischen Fluids aus Richtung des proximalen Kanalendes in Richtung des distalen Kanalendes, während ein Fördern des pharmazeutischen Fluids aus Richtung des distalen Kanalendes in Richtung des proximalen Kanalendes unterbunden ist. Das erfindungsgemäße Rückschlagventil ist also in Richtung des distalen Kanalendes fluidleitend und in Richtung des proximalen Kanalendes fluidundurchlässig ausgestaltet. Dies erlaubt somit ein Fördern und Applizieren eines pharmazeutischen Fluids aus einem mit dem Verbindungselement verbundenen Reservoir für das pharmazeutische Fluid durch die Vorrichtung und aus dem distalen Kanalende heraus, während ein Eindringen von Fluiden, insbesondere mit Bindegewebe versetzten Fluiden, in den Kanal proximal des Rückschlagventils verhindert wird. Dies verhindert eine, insbesondere vollständige, Kontamination und/oder Verstopfung der Vorrichtung und des mit der Vorrichtung verbundenen Reservoirs durch ein in das distale Kanalende eindringendes Fluid, wie beispielsweise Blut.

Distal zum Rückschlagventil, also näher am distalen Kanalende als das Rückschlagventil, ist im Kanal ein Dichtungselement angeordnet, welches das distale Kanalende solange fluidleitend verschließt, bis sich, ausgelöst durch eine ausreichend hohe Druckbeaufschlagung auf das im Kanal befindliche pharmazeutische Fluid aus Richtung des proximalen Kanalendes, ein Spalt im Dichtungselement fluidleitend öffnet. Ist die Druckbeaufschlagung nicht hoch genug, bleibt der Spalt fluidleitend verschlossen. Das pharmazeutische Fluids kann bei fortgesetzter Druckbeaufschlagung durch den fluidleitend geöffneten Spalt aus dem Kanal an eine gewünschte Stelle appliziert werden. Mit Beendigung der Druckbeaufschlagung schließt sich der zuvor ausgebildete fluidleitend geöffnete Spalt im Dichtungselement selbstständig wieder innerhalb weniger Augenblicke, beispielsweise innerhalb von Bruchteilen einer Sekunde, und das distale Kanalende ist wieder fluidleitend durch das Dichtungselement verschlossen. Dazu ist das Dichtungselement vorzugsweise aus einem elastischen Material ausgestaltet. Durch das Dichtungselement ist ein Einwachsen von Bindegewebe und ein Eindringen von Blut in die Vorrichtung unterbunden, weshalb die Vorrichtung funktionsfähig über mehrere Tage bis Monate im Patientenkörper implantiert sein kann und dabei eine wiederholte lokale Applikation eines pharmazeutischen Fluids an der gewünschten Stelle im Patienten ermöglicht ist. Ein weiterer Vorteil des Aufbaus der Vorrichtung ist, dass, selbst falls das distale Kanalende, beispielsweise durch Bindegewebe oder geronnenes Blut, fluidleitend verstopft sein sollte, diese Verstopfung durch die Druckbeaufschlagung und die darauffolgende Ausbringung des pharmazeutischen Fluids aus der Vorrichtung unter Druck beseitigt würde. Der Aufbau und die Funktionsweise der Vorrichtung ermöglicht somit eine "Selbstreinigung" der Verstopfung durch Gebrauch der Vorrichtung.

Das Dichtungselement kann dazu einen oder mehr als einen Spalt aufweisen, wobei der eine oder mehr als eine Spalt verschiedene Formen aufweisen kann. Aufgrund der einfachen Herstellungsweise ist ein einzelner, länglich ausgeformter Spalt bevorzugt.

Dabei kann die Vorrichtung auf zumindest zwei Arten betrieben werden. In einer ersten Art wird die Druckbeaufschlagung pulsartig ausgeführt, so dass bereits durch ein Applizieren einer kleinen Menge an pharmazeutischem Fluid, beispielsweise bis zu 2 Milliliter, aus der Vorrichtung heraus ein durch die Druckbeaufschlagung erzeugter Innendruck derart abnimmt, dass der Spalt wieder fluidleitend geschlossen wird. Dabei erreicht die Druckbeaufschlagung nur kurzfristig, also pulsartig, einen benötigten Grenzwert, um den Spalt reversibel fluidleitend auszuformen. In einer weiteren Art erfolgt eine kontinuierliche Druckbeaufschlagung auf das pharmazeutische Fluid, so dass dieses so lange aus dem distalen Kanalende austragbar ist, solange die Druckbeaufschlagung mit dem benötigten Grenzwert aufrechterhalten wird. Diese Art der Druckbeaufschlagung erlaubt somit eine kontinuierliche Applikation eines pharmazeutischen Fluids.

Sollte trotz des Dichtungselements mit Bindegewebe versetztes Fluid in das distale Kanalende eindringen, dient das Rückschlagventil als eine zweite Barriere für das eindringende Fluid. Dichtungselement und Rückschlagventil wirken also synergistisch zusammen, so dass eine Kontamination der Vorrichtung effektiv unterbunden wird. Dies erlaubt eine Implantation der Vorrichtung über einen im Vergleich zu einer Vorrichtung mit lediglich einer Barriere verlängerten Zeitraum.

Das Rückschlagventil kann unterschiedliche Bauformen aufweisen. In einer Ausgestaltungsform umfasst das Rückschlagventil eine Rückschlagklappe, welche ein Fördern des pharmazeutischen Fluids durch den Kanal nur in eine Förderrichtung erlaubt und in die entgegengesetzte Förderrichtung den Kanal fluidleitend verschließt.

Die Vorrichtung umfasst einen hohlzylinderförmigen Körper. Unter einem hohlzylinderförmigen Körper ist ein rohrartiges Strukturelement zu verstehen, welches einen Kanal mit einer dem Kanal zugewandten Innenfläche und einer dem Kanal abgewandten Außenfläche umgibt. Der Querschnitt des Körpers kann beliebige Formen annehmen. Aufgrund der einfachen Fertigung und der besseren Verankerungsmöglichkeit innerhalb eines Patienten ist der Querschnitt, und bevorzugt auch der Querschnitt des Kanals, im Wesentlichen kreisförmig ausgestaltet.

Der Kanal verläuft dabei von einem proximalen Körperende des Körpers durch den Körper bis zu einem distalen Körperende des Körpers. Dabei ist ein proximales Kanalende des Kanals dem proximalen Körperende und ein distales Kanalende des Kanals dem distalen Körperende zugewandt.

"Proximal" und "distal" dienen dabei lediglich der Bezeichnung der räumlich entgegengesetzten Enden der Vorrichtung, des Körpers oder anderer Struktureinheiten der Vorrichtung und erlauben keinen Rückschluss auf die Orientierung der in einen menschlichen Körper implantieren Vorrichtung. "Distal zu ..." und "proximal zu..." oder ähnliche Formulierungen drücken entsprechend lediglich die räumliche Anordnung zweier Struktureinheiten der Vorrichtung zueinander aus.

Die Außenfläche des Körpers weist zumindest abschnittsweise ein Außengewinde auf. Das Außengewinde dient der Verankerung der Vorrichtung in einem Gewebe, insbesondere einem Knochengewebe eines Patienten, durch Einschrauben der Vorrichtung in ein entsprechendes vorbereitetes Bohrloch im Gewebe, insbesondere im Knochengewebe. In einer Ausgestaltungsform erstreckt sich das Außengewinde über die gesamte axiale Erstreckung des Körpers, so dass der Körper über seine gesamte Länge in das entsprechende Bohrloch eingeschraubt werden kann. Das Außengewinde kann dabei auf einer Außenseite eines rohrartigen Elements angeordnet sein, welches den Körper manschettenartig umgibt. In einer bevorzugten Ausgestaltungsform ist die Außenfläche des Körpers selbst als Außengewinde ausgeformt.

Die Vorrichtung weist am proximalen Körperende ein fluidleitendes Verbindungselement auf, über das das proximale Kanalende reversibel fluidleitend mit einem Reservoir für das pharmazeutische Fluid verbindbar ist. In einer Ausgestaltungsform sind das Verbindungselement und das proximale Körperende einteilig ausgestaltet. In weiteren, bevorzugten Ausgestaltungsformen sind das Verbindungselement und das distale Körperende reversibel miteinander fluidleitend verbindbar ausgestaltet. Das Verbindungselement kann auf unterschiedliche Weise ausgestaltet sein, um das proximale Kanalende fluidleitend mit einem Reservoir für ein pharmazeutisches Fluid zu verbinden. In einer Ausgestaltungsform ist das Verbindungselement als Tülle ausgeformt, über die das proximale Kanalende mittels eines Schlauchs mit einem Reservoir fluidleitend verbindbar ist. In einer weiteren Ausgestaltungsform ist das Verbindungselement als Gewinde ausgeformt, welches über ein entsprechendes Gegenstück fluidleitend mit einem Reservoir verbindbar ist. In einer weiteren Ausgestaltungsform formt das Verbindungselement mit einem Reservoir eine Flanschverbindung. In einer weiteren Ausgestaltungsform formt das Verbindungselement über eine Schlauchkupplung mit einem Reservoir eine fluidleitende Verbindung, wobei das Verbindungselement die Kupplung oder den Nippel der Schlauchkupplung aufweisen kann.

Unter einem Reservoir werden alle Behältnisse verstanden, welche dazu geeignet sind, ein pharmazeutische Fluid zu lagern. Beispiele für Reservoire umfassen Beutel, Spritzen, Kolben, Ballons, Kanister und Ampullen, wobei Beutel, Ballons und Spritzen bevorzugt sind.

Die erfindungsgemäße Vorrichtung dient zur lokalen Applikation eines pharmazeutischen Fluids, insbesondere zur Behandlung entzündlicher Erkrankungen von Gelenken, wie die aktivierte Arthrose und die rheumatische Arthritis, über einen Zeitraum von mehreren Tagen bis mehreren Monaten. Ein pharmazeutisches Fluid enthält wenigstens einen pharmazeutischen Wirkstoff. Beispielsweise handelt es sich bei dem pharmazeutischen Fluid um wässrige oder nicht-wässrige Lösungen oder Suspensionen von pharmazeutischen Wirkstoffen.

In einer Ausgestaltungsform handelts es sich bei dem pharmazeutisches Fluid um Lösungen, welche wenigstens ein Antiphlogistikum, Cytostatikum und/oder Glucocorticoid enthalten. In einer weiteren Ausgestaltungsform enthalten die pharmazeutischen Fluide wenigstens einen desinfizierenden Bestandteil.

Weiterhin umfassen pharmazeutische Fluide auch Gase, Gasgemische und Lösungen von Gasen in Flüssigkeiten, wie beispielsweise Wasser.

Die zur Ausbildung des fluidleitend geöffneten Spalts im Dichtungselement benötigte Druckbeaufschlagung auf das pharmazeutische Fluid kann von unterschiedlichen Faktoren, wie beispielsweise dem Material des Dichtungselements, dem Durchmesser des Dichtungselements und der Länge des Dichtungselements abhängen.

Um ein kontrolliertes Applizieren des pharmazeutischen Fluids aus der Vorrichtung zu gewähren, ist eine Ausführungsform der Vorrichtung dadurch gekennzeichnet, dass sich der Spalt erst ab einer Druckbeaufschlagung von mindestens 5 N/cm² auf das pharmazeutische Fluid aus Richtung des proximalen Kanalendes reversibel fluidleitend im Dichtungselement öffnet, so dass das distale Kanalende fluidleitend geöffnet ist. Erreicht die Druckbeaufschlagung nicht diesen Grenzwert, bleibt der Spalt und damit das distale Kanalende fluidleitend verschlossen. Dies verhindert ein unbeabsichtigtes Austreten des pharmazeutischen Fluids aus der Vorrichtung in den Patienten, welches gesundheitliche Risiken nach sich ziehen könnte. Ein weiterer Vorteil ist, dass somit auch ein Eindringen von mit Gewebe, insbesondere mit Bindegewebe, versetzten Fluiden des Patienten nicht ohne Erreichen eines entsprechend entgegengesetzten Förderdrucks in Richtung des proximalen Kanalendes in die Vorrichtung durch das distale Kanalende zumindest erschwert ist. Ein Eindringen dieser körpereigener Fluide könnte eine Kontamination der Vorrichtung oder ein Verstopfen der Vorrichtung nach sich ziehen. Damit ein Applizieren kontrolliert, auch in kleinen Mengen und ohne das Risiko einer unbeabsichtigten Zerstörung in der Vorrichtung, wie beispielsweise einer Rissbildung im Körper oder im Dichtungselement, stattfinden kann, ist es bevorzugt, dass zur fluidleitenden Öffnung des Spalts die Druckbeaufschlagung nicht mehr als 150 N/cm² betragen muss.

Das Rückschlagventil kann unterschiedliche Bauformen aufweisen. In einer Ausgestaltungsform umfasst das Rückschlagventil eine Rückschlagklappe, welche ein Fördern des pharmazeutischen Fluids durch den Kanal in nur eine Förderrichtung, insbesondere in Richtung des distalen Kanalendes, erlaubt und in die entgegengesetzte Förderrichtung, insbesondere in Richtung des proximalen Kanalendes, den Kanal fluidleitend verschließt. In einer weiteren Ausgestaltungsform ist das Rückschlagventil als Tellerrückschlagventil ausgebildet.

Eine Ausführungsform der Vorrichtung ist dadurch gekennzeichnet, dass das Rückschlagventil ein Kugelrückschlagventil umfassend eine Kugel und ein Rückstellelement ist. Bei einem Kugelrückschlagventil wird ein Leitungsmittel fluidleitend durch eine Kugel verschlossen, welche durch die Schwerkraft, oder wie erfindungsgemäß vorgesehen, durch ein Rückstellelement gegen das Leitungsmittel gehalten wird um dieses fluidleitend zu verschließen. Ein Fluid, welches durch das Leitungsmittel gegen die Kugel und das auf die Kugel wirkende Rückstellelement gefördert wird, kann, falls der Förderdruck den Druck des Rückstellelements auf die Kugel überwiegt, das Kugelrückschlagventil fluidleitend öffnen. Aus der entgegengesetzten Förderrichtung, also aus Richtung des Rückstellelements in Richtung des Leitungsmittels, bleibt das Kugelrückschlagventil fluidleitend durch die Kugel verschlossen. Ein Vorteil eines Kugelrückschlagventils ist die einfache, kostengünstige Bauweise und eine geringe Anfälligkeit für Funktionsstörungen.

Das Rückstellelement kann dabei auf unterschiedliche Weisen ausgestaltet sein, um die Kugel gegen das Leitungsmittel zu drücken und das Kugelrückschlagventil fluidleitend zu verschließen. In einer Ausgestaltungsform umfasst das Rückstellelement eine Feder, beispielsweise eine Spiralfeder oder Blattfeder, beispielsweise aus einem Metall. In einer weiteren Ausgestaltungsform umfasst das Rückstellelement ein elastisches Polymerelement, insbesondere ein rohrartig oder blattfederartig ausgestaltetes Polymerelement.

Das Rückstellelement kann als separates Bauteil vorliegen oder mit weiteren Bauteilen der Vorrichtung verbunden sein.

Eine Ausführungsform der Vorrichtung ist dadurch gekennzeichnet, dass das Rückstellelement und das Dichtungselement einteilig ausgestaltet sind. Dies reduziert die Anzahl der Bauteile der Vorrichtung, was die Fertigung derselben vereinfacht und ebenso eine Verringerung der Anfälligkeit von Funktionsstörungen zur Folge hat. Da das Dichtungselement bevorzugt aus einem elastischen Material gefertigt ist, erlaubt die Ausgestaltung des dem Rückschlagventil, insbesondere der Kugel des Kugelrückschlagventils, zugewandten Ende des Dichtungselements als Rückstellelement, beispielsweise als elastisches Rohrstück oder als elastische Blattfederelemente, eine einfache und kostengünstige Konstruktion der Vorrichtung.

Um das pharmazeutische Fluid von eventuell vorhandenen Mikroorganismen zu befreien, ist eine Ausführungsform der Vorrichtung dadurch gekennzeichnet, dass im Kanal ein Filterelement angeordnet ist.

Das Filterelement kann an unterschiedlichen Stellen innerhalb des Kanals angeordnet sein.

Um eine vollständige Kontamination der Vorrichtung, beispielsweise durch Mikroorganismen, zu verhindern, ist eine Ausführungsform der Vorrichtung dadurch gekennzeichnet, dass das Filterelement im Kanal proximal zum Rückschlagventil, also näher am proximalen Kanalende als das Rückschlagventil, angeordnet ist. Das pharmazeutische Fluid durchläuft in dieser Ausführungsform der Vorrichtung beim Fördern durch den Kanal aus Richtung des proximalen Kanalendes zuerst das Filterelement, gefolgt vom Rückschlagventil und schließlich das Dichtungselement. Somit erfolgt beim Applizieren des pharmazeutischen Fluids eine Filtration durch das Filterelement bevor das pharmazeutische Fluid das Rückschlagventil und das Dichtungselement erreicht.

Das Filterelement kann unterschiedlich ausgestaltet sein. In einer Ausgestaltungsform umfasst das Filterelement ein Vlies, insbesondere ein Glasfaservlies. In einer weiteren Ausgestaltungsform umfasst das Filterelement ein Fasermaterial, insbesondere ein Polyester-, Metall- und/oder ein Zellulosefasermaterial. In einer weiteren Ausgestaltungsform umfasst das Filterelement ein Drahtgewebe, insbesondere ein Edelstahldrahtgewebe.

Eine Ausführungsform der Vorrichtung ist dadurch gekennzeichnet, dass das Filterelement eine mikroporöse Filterplatte umfasst, insbesondere dass das Filterelement eine mikroporöse Filterplatte ist.

Die mikroporöse Filterplatte kann Poren mit unterschiedlich großen durchschnittlichen Porendurchmessern aufweisen. Unter einem durchschnittlichen Porendurchmesser ist das arithmetische Mittel der Porendurchmesser der Poren der Filterplatte zu verstehen.

Eine Ausführungsform der Vorrichtung ist dadurch gekennzeichnet, dass die mikroporöse Filterplatte Poren mit einem durchschnittlichen Porendurchmesser von kleiner 40 µm, bevorzugt kleiner als 5 µm, weiter bevorzugt von kleiner als 1 µm, aufweist. Dies erlaubt eine gute Reinigung des pharmazeutischen Fluids. Um trotz der guten Filtrationsleistung ein reibungsloses Fördern des pharmazeutischen Fluids durch die Vorrichtung hindurch zu gewährleisten, ist es bevorzugt, dass der durchschnittlichen Porendurchmesser nicht kleiner als 0,05 µm ist.

Um ein Austragen des Dichtungselements aus dem distalen Kanalende während des Applizierens eines pharmazeutischen Fluids mittels der Vorrichtung zu verhindern, kann das Dichtungselement auf unterschiedliche Weise im Kanal befestigt werden. In einer Ausgestaltungsform ist das Dichtungselement mittels eines Klebstoffs innerhalb des Kanals fixiert.

Eine Ausgestaltungsform der Vorrichtung ist dadurch gekennzeichnet, dass im Kanal distal zum Dichtungselement, also näher am distalen Kanalende als das Dichtungselement, eine hohlzylinderförmige Hülse angeordnet ist, um ein Austragen des Dichtungselements aus dem distalen Kanalende beim Applizieren des pharmazeutischen Fluids zu unterbinden. Um die hohlzylinderförmige Hülse selbst im Kanal zu fixieren, kann diese mit der Innenfläche des Körpers mittels einer Nut-Feder-Verbindung form- und/oder kraftschlüssig zusammenwirken und so ein Austragen verhindern.

Das Verbindungselement und der hohlzylinderförmige Körper können auf unterschiedliche Weise miteinander verbunden sein um das Verbindungelement und das proximale Kanalende fluidleitend zu verbinden. In einer Ausgestaltungsform sind das Verbindungselement und der Körper einteilig ausgestaltet.

In weiteren Ausgestaltungsformen sind das Verbindungselement und der Körper mittels einer Klebeverbindung, einer Bajonettverbindung oder einer Gewindeverbindung miteinander verbunden.

Eine Ausführungsform der Vorrichtung ist dadurch gekennzeichnet, dass das proximale Kanalende mehrkantförmig, insbesondere sechskantförmig, ausgebildet ist und das Verbindungselement mit einem mehrkantförmigen, insbesondere sechskantförmigen, Verbindungselementabschnitt ausgestattet ist, um das Verbindungselement mit dem proximalen Kanalende reversibel fluidleitend durch Einstecken des Verbindungselementabschnitts in das proximale Kanalende zu verbinden.

Das Einstecken, also ein Einschieben des Verbindungselementabschnitts in das proximale Kanalende, erlaubt eine sichere, einfache und kostengünstige Möglichkeit der Befestigung des Verbindungselements mit dem proximalen Kanalende. Das proximale Kanalende und der Verbindungselementabschnitt wirken nach dem Einstecken form- und/oder kraftschlüssig zusammen, lassen sich aber bei Bedarf auch wieder durch Ziehen an dem Verbindungselement voneinander lösen. Dieser Vorgang ist beliebig häufig wiederholbar. Um die beiden Bauteile sicher miteinander fluidleitend zu verbinden, weisen das Verbindungselement und das proximale Kanalende vorzugsweise die gleiche Anzahl an Kanten und zueinander passende Kantendimensionen auf, so dass diese "Schlüssel-Schloss"-artig zusammengeschoben werden können. Ein Vorteil dabei ist, dass so ein unerwünschtes axiales Drehen der beiden Bauteile zueinander verhindert wird und die Vorrichtung ist einer gewünschten Ausrichtung mit einem Reservoir für ein pharmazeutisches Fluid verbindbar ist. Ein weiterer Vorteil ist, dass durch die mehrkantförmige Ausgestaltung des proximalen Kanalendes die Vorrichtung mittels eines dazu passenden mehrkantförmigen Werkzeugs, insbesondere eines Schlüssels, in ein entsprechend vorbereitetes Bohrloch im Knochengewebe eines Patienten eingeschraubt werden kann.

Um ein vollständiges Einstecken des mehrkantförmigen Verbindungselementabschnitts in das proximale Kanalende zu erlauben, ist es bevorzugt, dass das mehrkantförmig ausgestaltete proximale Kanalende zumindest die gleiche axiale Erstreckung wie der mehrkantförmig ausgestaltete Verbindungselementabschnitt aufweist.

Eine Ausführungsform der Vorrichtung ist dadurch gekennzeichnet, dass das mehrkantförmig ausgebildete proximale Kanalende eine größere axiale Erstreckung aufweist als der mehrkantförmige Verbindungselementabschnitt, so dass nach einem Entfernen eines Teils des proximalen Kanalendes, insbesondere durch Absägen oder Abschneiden, das Verbindungselement mit einem verbleibenden Teil des proximalen Kanalendes fluidleitend durch Einstecken des Verbindungselementabschnitts in das verbleibende proximale Kanalende verbindbar ist.

Die Vorrichtung kann auf unterschiedliche Weise in das Knochengewebe eines Patienten implantiert werden. Um eine möglichst hohe Beweglichkeit des Gelenks und so einen möglichst natürlichen, möglichst schmerzfreien Bewegungsablauf zu gewährleisten, ist es bevorzugt die Vorrichtung möglichst vollständig im Knochengewebe zu versenken. Je nach Größe des Patienten und der Dicke des vorhandenen Knochengewebes, kann es nötig sein, die Vorrichtung für ein möglichst vollständiges Versenken im Knochengewebe zu kürzen.

Ist die axiale Erstreckung, also die Länge, des mehrkantförmig ausgestalteten proximalen Kanalendes größer als die axiale Erstreckung des mehrkantförmig ausgestalteten Verbindungselementabschnitts, kann das proximale Kanalende, beispielsweise durch Abschneiden oder Absägen, gekürzt werden, wobei nach dem Kürzen des proximalen Kanalendes der Verbindungselementabschnitt immer noch vollständig in das proximale Kanalende einschiebbar ist, um das Verbindungselement fluidleitend mit dem Kanal zu verbinden.

In einer Ausführungsform der Vorrichtung umfasst der Körper ein Metall, ein Polymer oder ein Metall und ein Polymer, insbesondere besteht der Körper aus einem Metall, einem Polymer oder einem Metall und einem Polymer. Beispiele für Polymere umfassen Polyamide, Polyester, Polyketone, Polymethacrylate und deren Copolymere. Beispiels für Metalle umfassen reine Metalle wie Aluminium und Titan oder Metalllegierungen wie Edelstähle, insbesondere Edelstahl 1.4404, oder Titanlegierungen, wie TiAl₆V₄.

Um eine sachgerechte und zielgerichtete Applikation an der gewünschten Stelle innerhalb eines Patienten zu kontrollieren, ist eine Ausführungsform der Vorrichtung dadurch gekennzeichnet, dass zumindest ein Teilbereich der Vorrichtung, insbesondere der hohlzylinderförmige Körper der Vorrichtung, einen Röntgenopaker aufweist. Durch einen Röntgenopaker kann die richtige Positionierung der Vorrichtung innerhalb des Patienten über bildgebende Verfahren mittels Röntgenstrahlung (X-ray) sichtbar gemacht werden. Beispiele für Röntgenopaker sind Bariumsulfat, Zirkondioxid und Kalziumcarbonat.

Ein weiterer Gegenstand der Offenbarung betrifft ein Verfahren zur Applikation eines pharmazeutischen Fluids mittels einer Vorrichtung nach einer der vorhergehenden Ausführungsformen, aufweisend die folgenden Schritte:
a. Implantieren der Vorrichtung;
b. Fluidleitendes Verbinden der Vorrichtung mit einem Reservoir für das pharmazeutischen Fluids;
c. Aufbau eines Förderdrucks auf das pharmazeutische Fluid aus Richtung des proximalen Kanalendes von größer 5 N/cm²;
d. Fluidleitendes Öffnen des Spalts im Dichtungselements durch Einwirkung des Förderdrucks;
e. Ausbringen des pharmazeutischen Fluids aus dem fluidleitend geöffneten Spalt;
f. Fluidleitendes Verschließen des Spalts durch Reduktion des Förderdrucks auf 5 N/cm² oder weniger.

Das Implantieren der Vorrichtung in den Patienten kann auf unterschiedliche Weise erfolgen. In einer bevorzugten Ausgestaltungsform erfolgt das Implantieren durch Einschrauben der Vorrichtung in ein dafür vorbereitetes Bohrloch im Knochengewebe des Patienten. Die Implantation erfolgt bevorzugt in räumlicher Nähe einer Gelenkkapsel, so dass mittels der Vorrichtung appliziertes pharmazeutisches Fluid den entsprechenden Gelenkraum erreichen kann. Um Komplikationen und potenzielle Infektionen des Gelenks zu verhindern, wird die Vorrichtung vorzugsweise so implantiert, dass die Gelenkkapsel nicht perforiert wird. Beispielsweise kann die Vorrichtung zur Behandlung eines Kniegelenks in eine Bohrung im distalen Femur eingesetzt werden, wobei die Bohrung im interkondylären Raum endet. Um ein sicheres Fixieren der Vorrichtung im Knochengewebe zu erreichen, wird ein Durchmesser der Bohrung so gewählt, dass die Vorrichtung im Wesentlichen spaltfrei in das Bohrloch eingeschraubt werden kann.

Die Länge des Bohrlochs kann dabei röntgenographisch (X-ray) bestimmt werden, so dass die Vorrichtung bei Bedarf vor der Implantation gekürzt werden kann.

Vorzugsweise wird die Vorrichtung so weit in das Bohrloch eingeschraubt, dass das proximale Körperende dicht unterhalb, beispielsweise 1 mm unterhalb, der entsprechenden Gelenkfläche endet. Dies gewährleistet einen weitgehend normalen Bewegungsablauf des entsprechenden Gelenks.

Nach erfolgter Implantation wird die Vorrichtung fluidleitend mit einem Reservoir für das pharmazeutische Fluid verbunden. Die fluidleitende Verbindung kann auf unterschiedliche Weise realisiert werden, wobei es bevorzugt ist, die Vorrichtung und das Reservoir mittels eines flexiblen Schlauchs fluidleitend zu verbinden. Um den Schlauch mit dem Reservoir fluidleitend zu verbinden, kann beispielsweise ein Adapter, insbesondere ein Luer-System mit Abdeckkappe, verwendet werden. Pharmazeutische Fluide können dadurch bei Bedarf jederzeit mit einer einfachen Spritze in den zu behandelnden intraartikulären Raum eingebracht werden.

Um eine Kontamination mit Mikroorganismen zu verhindern, ist es bevorzugt den Schlauch mit einem Sterilfilter auszustatten.

Um das pharmazeutische Fluid aus der Vorrichtung, insbesondere dem distalen Kanalende, zu applizieren, wird ein Förderdruck auf das pharmazeutische Fluid aufgebaut, welches aus Richtung des proximalen Kanalendes in Richtung des distalen Kanalendes wirkt. Der Förderdruck fördert das pharmazeutische Fluid aus dem Reservoir in das proximale Kanalende der Vorrichtung, durch das Rückschlagventil und das Dichtungselement und letztendlich aus dem distalen Kanalende heraus. Um das Rückschlagventil und insbesondere auch das Dichtungselement zu passieren, wird ein Förderdruck auf das pharmazeutische Fluid von mindestens 5 N/cm² ausgeübt. Dieser Förderdruck reicht aus, um den Spalt im Dichtungselement reversibel fluidleitend zu öffnen und das pharmazeutische Fluid so aus der Vorrichtung, insbesondere dem distalen Kanalende, ausfließen zu lassen.

Der Förderdruck auf das pharmazeutische Fluid zur Ausbildung des fluidleitenden geöffneten Spalts im Dichtungselement kann auf unterschiedliche Arten ausgeübt werden. In einer Ausgestaltungsform wird der Förderdruck mittels einer separaten Pumpe, beispielsweise einer Peristaltikpumpe, welche auf einen als fluidleitende Verbindung zwischen Vorrichtung und Reservoir eingesetzten Schlauch einwirkt, ausgeübt. In einer weiteren Ausgestaltungsform ist das Reservoir für das pharmazeutische Fluid eine Spritze und der Förderdruck auf das pharmazeutische Fluid wird mittels eines zur Spritze gehörenden Spritzenkolbens ausgeübt.

Wird der Förderdruck auf 5 N/cm² oder weniger reduziert, schließt sich der Spalt im Dichtungselement selbstständig innerhalb weniger Augenblicke, beispielsweise innerhalb einer Sekunde oder kürzer, und das Applizieren des pharmazeutischen Fluids wird beendet.

Die für die Vorrichtung offenbarten Merkmale sind auch für das Verfahren offenbart und umgekehrt.

### Figuren

Die Erfindung wird im Folgenden durch Figuren weiter beispielhaft illustriert. Die Erfindung ist nicht auf die Figuren beschränkt.

### Es zeigen

- Fig. 1: einen schematischen Längsschnitt einer Vorrichtung zum Applizieren eines pharmazeutischen Fluids,
- Fig. 2: die Vorrichtung aus Figur 1 in einer perspektiven Seitenansicht eines teilweisen schematischen Längsschnitts einer,
- Fig. 3: die Vorrichtung aus den Figuren 1 und 2 mit gefördertem pharmazeutischen Fluid,
- Fig. 4: die Vorrichtung aus den Figuren 1 bis 3 beim Applizieren des pharmazeutischen Fluids,
- Fig. 5: die Vorrichtung aus den Figuren 1 bis 4, fluidleitend verbunden mit einem Reservoir für ein pharmazeutisches Fluid, und
- Fig. 6: ein Verfahren zur Applikation eines pharmazeutischen Fluids.

### Beschreibung der Figuren

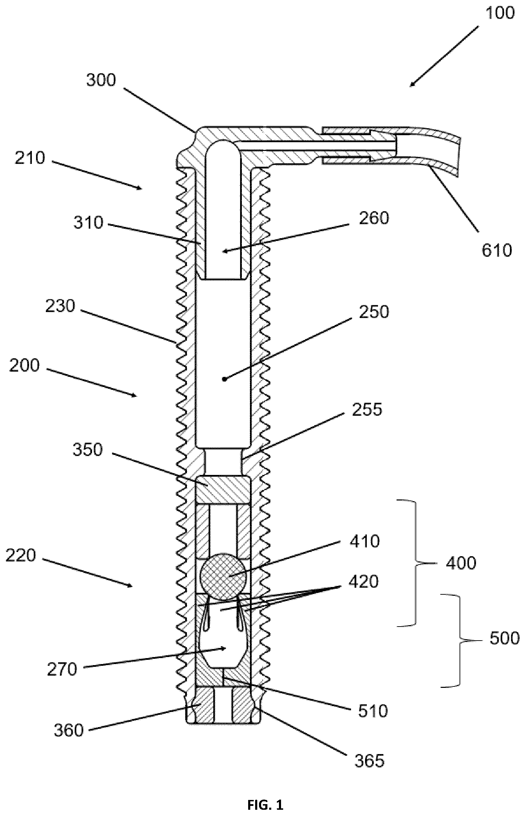

**Figur 1** zeigt einen schematischen Längsschnitt einer beispielhaften Ausführung einer Vorrichtung 100 zum Applizieren eines pharmazeutischen Fluids (in Figur 1 nicht gezeigt). Die Vorrichtung 100 umfasst einen hohlzylinderförmigen Körper 200 mit einem proximalen Körperende 210 und einem dem proximalen Körperende 210 axial gegenüberliegendem distalen Körperende 220. Eine Außenfläche des Körpers 200 ist als ein Außengewinde 230 ausgeformt. In der gezeigten Ausführungsform der Vorrichtung 100 erstreckt sich das Außengewinde 230 über nahezu die gesamte Länge der Vorrichtung 100. In weiteren, nicht gezeigten Ausführungsformen der Vorrichtung 100 erstreckt sich das Außengewinde 230 nicht nahezu über die gesamte Länge, sondern beispielsweise lediglich über 50% der gesamten Länge der Vorrichtung 100. Das Außengewinde 230 dient dem Einschrauben der Vorrichtung 100 in ein Bohrloch im Knochen eines Patienten.

Axial durch den hohlzylinderförmigen Körpers 200 verläuft ein fluidleitender Kanal 250, wobei sich der Kanal 250 von einem dem proximalen Körperende 210 zugewandten proximalen Kanalende 260 durch den gesamten Körper 200 bis zu einem dem distalen Körperende 220 zugewandten distalen Kanalende 270 erstreckt. Das proximale Kanalende 260 ist fluidleitend mit einem Verbindungselement 300 verbunden, über das die Vorrichtung 100 mit einem Reservoir für ein pharmazeutisches Fluid (beides nicht gezeigt in Figur 1) fluidleitend verbindbar ist. Um das Verbindungselement 300 fluidleitend mit dem proximalen Kanalende 260 zu verbinden, weist das Verbindungselement 300 einen Verbindungselementabschnitt 310 auf, welcher in das proximale Kanalende 260 eingesteckt ist. In der gezeigten Ausführungsform ist das Verbindungselement 300 als Tülle ausgeformt, so dass ein Reservoir für ein pharmazeutisches Fluid mittels eines Schlauchs 610 (lediglich abschnittsweise gezeugt) fluidleitend mit der Vorrichtung 100 verbindbar ist.

Im Kanal 250 ist ein Rückschlagventil 400 in Form eines Kugelrückschlagventils umfassend eine Kugel 410 und ein Rückstellelement 420 in Form vierer elastischer, blattfederartig ausgeformter Elemente, angeordnet (nur drei der Elemente sind gezeigt, das vierte befindet sich außerhalb der Zeichenebene). Das Rückstellelement 420 übt eine Kraft auf die Kugel 410 aus, so dass diese das Rückschlagventil 400 fluidleitend verschließt. Das Rückschlagventil 400 ist dabei so angeordnet, dass ein Fluid aus Richtung des proximalen Kanalendes 260 kommend die Kugel 410 gegen die Kraft des Rückstellelements 420 in Richtung des distalen Kanalendes 270 verschieben kann, so dass das Rückschlagventil 400 fluidleitend geöffnet wird. In die entgegengesetzte Richtung, also vom distalen Kanalende 270 in Richtung des proximalen Kanalendes 260 verbleibt das Rückschlagventil 400 fluidleitend verschlossen und kann nicht zerstörungsfrei geöffnet werden.

Distal zum Rückschlagventil 400 ist innerhalb des Kanals 250 ein Dichtungselement 500 umfassend einen fluidleitend verschlossenen Spalt 510 angeordnet. In der gezeigten Ausführungsform der Vorrichtung 100 sind das Rückstellelement 420 und das Dichtungselement 500 einteilig ausgestaltet.

Um das Dichtungselement 500, und damit auch das Rückschlagventil 500, gegen ein unbeabsichtigtes Austragen aus dem Kanal 250 beim Applizieren eines pharmazeutischen Fluids zu schützen, ist distal zum Dichtungselement 500 eine Hülse 360 angeordnet, welche über eine Feder-Nut-Verbindung 365 mit dem Körper 200 im Kanal 250 fixiert ist.

Proximal des Rückschlagventils 400 ist im Kanal 250 ein Filterelement 350 in Form einer mikroporösen Filterplatte angeordnet, um ein durch die Vorrichtung 100 gefördertes pharmazeutisches Fluid von Mikroorganismen zu reinigen.

Als Gegenstück zu der Hülse 260 weist der Kanal 250 eine Kanalverengung 255 auf, so dass das Filterelement 350, das Rückschlagventil 400 und das Dichtungselement 500 zwischen Hülse 360 und Kanalverengung 255 fixiert sind.

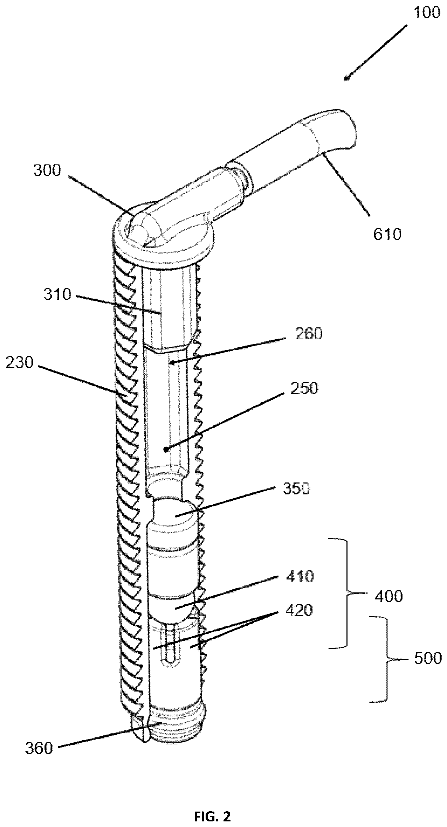

**Figur 2** zeigt die Vorrichtung 100 aus Figur 1 in einer perspektivischen Seitenansicht eines teilweisen schematischen Längsschnitts. Zur Ausgestaltung und Anordnung der einzelnen Strukturmerkmale wird auf die Ausführungen zu Figur 1 verwiesen. In der in Figur 2 dargestellten Perspektive der Vorrichtung 100 ist erkennbar, dass der Verbindungselementabschnitt 310 und das proximale Kanalende 260 mehrkantförmig, insbesondere sechskantförmig, ausgestaltet sind, so dass der Verbindungselementabschnitt 310 reversibel verdrehungssicher in das proximale Kanalende 260 einsteckbar ist.

Das mehrkantförmig ausgestaltete proximale Kanalende 260 weist eine größere axiale Erstreckung als der mehrkantförmig ausgestaltete Verbindungselementabschnitt 310 auf. Dies erlaubt ein Kürzen des proximalen Kanalendes 260, beispielsweise durch Absägen oder Abschneiden desselbigen, und ein fluidleitendes Verbinden des Verbindungselementabschnitts 310 mit dem nach dem Kürzen verbleibenden Rest des proximalen Kanalendes 260. Die so in ihrer Gesamtlänge verkürzte Vorrichtung 100 lässt damit auch ein Implantieren in Knochengewebe zu, welches vor der Kürzung eine unzureichende Dicke aufgewiesen hätte.

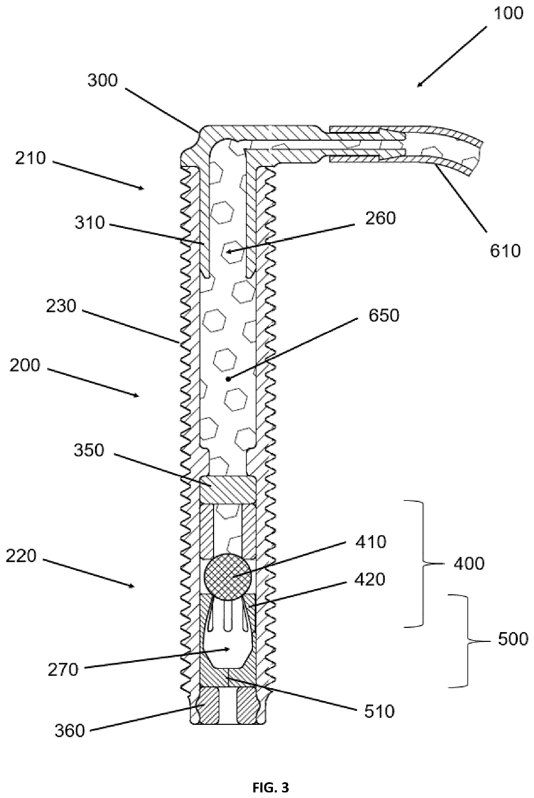

**Figur 3** zeigt die Vorrichtung 100 aus den Figuren 1 und 2, wobei ein pharmazeutisches Fluid 650 über den Schlauch 610 in das proximale Kanalende 260 eingeleitet wurde. Das pharmazeutische Fluid 650 füllt den Kanal 250 vom proximalem Endes 260 bis zur Kugel 410 aus. Um das pharmazeutische Fluid 650 weiter in Richtung des distalen Kanalendes 270 zu fördern, muss ein Förderdruck auf das pharmazeutische Fluid 650 aufgebaut werden, welcher zumindest die auf die Kugel 40 wirkende Kraft des Rückstellelements 420 überwindet und somit das Rückschlagventil 400 fluidleitend öffnet.

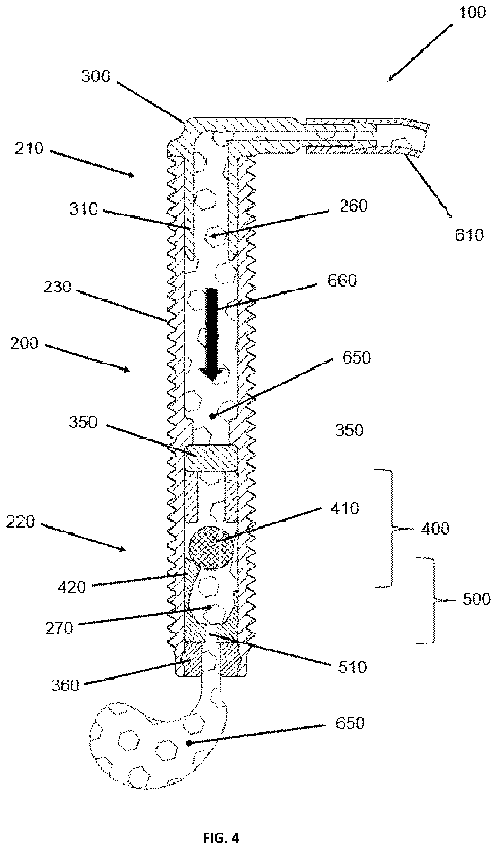

**Figur 4** zeigt die Vorrichtung 100 aus den Figuren 1 bis 3, wobei auf das pharmazeutische Fluid 650 ein ausreichend hoher Förderdruck 660 (symbolisiert als Pfeil) aus Richtung des proximalen Kanalendes 260 ausgeübt wird, um die Kugel 410 gegen die Rückstellkraft des Rückstellelements 420 in Richtung des distalen Kanalendes 270 zu verschieben. Dabei wird das Rückstellelement 420 reversibel verformt und das Rückschlagventil 400 ist fluidleitend geöffnet. Weiterhin ist der Förderdruck 660 ausreichend hoch, um auch den Spalt 510 des Dichtungselements 500 reversibel fluidleitend zu öffnen. Dies erlaubt ein Applizieren des pharmazeutischen Fluids 650 aus dem distalen Kanalende 270. Wird der Förderdruck 660 reduziert, so dass sich der Spalt 510, das Rückschlagventil 400 oder der Spalt 510 und das Rückschlagventil 400 wieder reversibel fluidleitend verschließt, stoppt die Applikation des pharmazeutischen Fluids 650 innerhalb weniger Augenblicke, beispielsweise innerhalb einer Sekunde oder weniger.

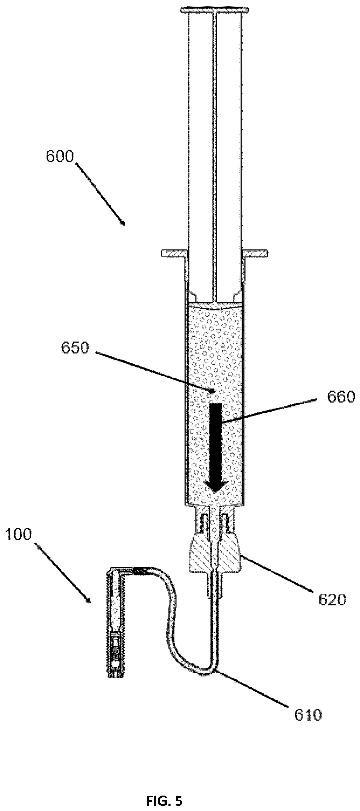

**Figur 5** zeigt die Vorrichtung 100 aus den Figuren 1 bis 4 fluidleitend verbunden mit einem Reservoir 600 für ein pharmazeutisches Fluid 650 in Form einer Spritze. Die Vorrichtung 100 ist dabei über einen Schlauch 610 und einen Adapter 620 in Form eines Luer-Systems fluidleitend mit dem Reservoir 600 verbunden. Durch ein Betätigen der Spritze kann ein Förderdruck 660 auf das pharmazeutische Fluid 650 aufgebaut werden und dieses durch die Vorrichtung 100 an eine gewünschte Stelle appliziert werden.

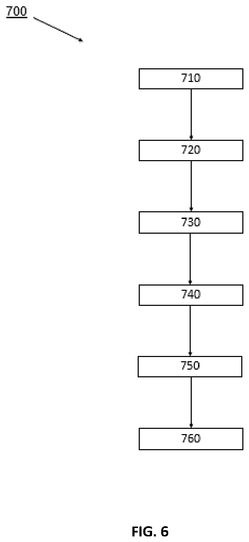

**Figur 6** zeigt ein Flussdiagramm eines Verfahrens zur Applikation eines pharmazeutischen Fluids 650 mittels der Vorrichtung 100 umfassend die Schritte 710 bis 760. Die Vorrichtung 100 umfasst den hohlzylinderförmigen Körper 200, welcher den Kanal 250 umgibt, der sich vom proximalen Körperende 210 zum distalen Körperende 220 des Körpers 200 durch den Körper 200 erstreckt, wobei die Außenfläche des Körpers 200 zumindest teilweise das Außengewinde 230 aufweist, das am proximalen Körperende 210 angeordnete Verbindungselement 300 über das proximale Kanalende 260 des Kanals 250 fluidleitend mit einem Reservoir 600 für das pharmazeutische Fluid 650 reversibel verbindbar ist, wobei das im Kanal 250 angeordnete Rückschlagventil 400, welches in Richtung des proximalen Kanalendes 260 fluidundurchlässig ausgestaltet ist, und das im Kanal 250 distal zum Rückschlagventil 400 angeordnete Dichtungselement 500, welches das distale Kanalende 270 des Kanals 250 fluidleitend verschließt und bei einer Druckbeaufschlagung 660 auf das pharmazeutische Fluid 650 aus Richtung des proximalen Kanalendes 260 reversibel der Spalt 510 fluidleitend öffenbar ist, so dass das pharmazeutische Fluid (650) aus dem distalen Kanalende (270) applizierbar ist.

In Schritt 710 erfolgt ein Implantieren der Vorrichtung 100, insbesondere in ein Knochengewebe eines Patienten in räumlicher Nähe eines Gelenks, beispielsweise in eine Kondyle eines entsprechenden Knochens.

In Schritt 720 wird die Vorrichtung 100 fluidleitend mit einem Reservoir 600 für das pharmazeutische Fluid 650 verbunden. Dies kann beispielsweise über einen Schlauch 610 geschehen. Das fluidleitende Verbinden 720 kann bereits vor oder erst nach erfolgter Implantation in Schritt 710 erfolgen, wobei nach erfolgter Implantation bevorzugt ist.

In Schritt 730 wird ein Förderdruck 660 von mindestens 5 N/cm² auf das pharmazeutische Fluid 650 aus Richtung des proximalen Kanalendes 260 aufgebaut. Dieser Förderdruck 660 reicht aus, um das Rückschlagventil 400 und das Dichtungselement 500, insbesondere den Spalt 510 im Dichtungselement 500, fluidleitend zu öffnen. Ist der Förderdruck 660 geringer als 5 N/cm², bleibt zumindest der Spalt 510 im Dichtungselement 500 fluidleitend geschlossen und das pharmazeutische Fluid 650 wird nicht aus der Vorrichtung 100, insbesondere dem distalen Kanalende 270der Vorrichtung 100, ausgeleitet. Dieser Grenzwert des Förderdrucks 660 stellt sicher, dass es nicht zu einer unbeabsichtigten und unkontrollierten Applikation des pharmazeutischen Fluids 610 aus der Vorrichtung 100 kommt, welche negative Auswirkungen für den Patienten nach sich ziehen könnte.

Konzertiert mit dem fluidleitenden Öffnen des Spalts 510 kommt es in Schritt 750 zum Ausbringen des pharmazeutischen Fluids 650 aus dem selbigen. Das Ausbringen des pharmazeutischen Fluids 650 setzt sich fort, solange der Förderdruck 660 über dem Grenzwert von 5 N/cm² liegt oder das pharmazeutische Fluids 650 vollständig ausgetragen ist.

In Schritt 760 wird der Förderdruck 660 unter den Grenzwert von 5 N/cm² abgesengt, wodurch sich zumindest der Spalt 510 wieder selbstständig fluidleitend verschließt und die Applikation des pharmazeutischen Fluids 650 beendet wird.

Das Verfahren 700 kann beliebig häufig durchgeführt werden.

### Bezugszeichen

- 100: Vorrichtung
- 200: hohlzylinderförmiger Körper
- 210: proximales Körperende
- 220: distales Körperende
- 230: Außengewinde
- 250: Kanal
- 255: Kanalverengung
- 260: proximales Kanalende
- 270: distales Kanalende
- 300: Verbindungselement
- 310: Verbindungselementabschnitt
- 350: Filterelement
- 360: Hülse
- 365: Feder-Nut-Verbindung
- 400: Rückschlagventil
- 410: Kugel
- 420: Rückstellelement
- 500: Dichtungselement
- 510: Spalt
- 600: Reservoir
- 610: Schlauch
- 620: Adapter
- 650: pharmazeutisches Fluid
- 660: Druckbeaufschlagung
- 700: Verfahren zur Applikation eines pharmazeutischen Fluids
- 710: Implantieren
- 720: fluidleitendes Verbinden
- 730: Aufbau eines Förderdrucks
- 740: fluidleitendes Öffnen
- 750: Ausbringen
- 760: fluidleitendes Verschließen

## Patentansprüche

1. Vorrichtung (100) zur Applikation eines pharmazeutischen Fluids (650) in eine Gelenkkapsel umfassend
einen hohlzylinderförmigen Körper (200), der einen Kanal (250) umgibt, der sich von einem proximalen Körperende (210) zu einem distalen Körperende (220) des Körpers (200) durch den Körper (200) erstreckt,
wobei eine Außenfläche des Körpers (200) zumindest teilweise ein Außengewinde (230) aufweist,
ein am proximalen Körperende (210) angeordnetes Verbindungselement (300), über das ein proximales Kanalende (260) des Kanals (250) reversibel fluidleitend mit einem Reservoir (600) für das pharmazeutische Fluid (650) verbindbar ist, und
ein im Kanal (250) angeordnetes Rückschlagventil (400), welches in Richtung des proximalen Kanalendes (260) fluidundurchlässig ausgestaltet ist,
**gekennzeichnet durch**
ein im Kanal (250) distal zum Rückschlagventil (400) angeordnetes Dichtungselement (500), welches ein distales Kanalende (270) des Kanals (250) fluidleitend verschließt und welches einen Spalt (510) aufweist, welcher bei einer Druckbeaufschlagung (660) auf das pharmazeutische Fluid (650) aus Richtung des proximalen Kanalendes (260) reversibel fluidleitend öffenbar ist, so dass das pharmazeutische Fluid (650) aus dem distalen Kanalende (270) applizierbar ist und wobei das Rückschlagventil (400) ein Kugelrückschlagventil umfassend eine Kugel (410) und ein Rückstellelement (420) ist.

2. Vorrichtung (100) nach Anspruch 1, **wobei** sich der Spalt (510) bei einer Druckbeaufschlagung (660) von größer als 5 N/cm² reversibel fluidleitend öffnet.

3. Vorrichtung (100) nach Anspruch 1 oder 2, **wobei** das Rückstellelement (420) und das Dichtungselement (500) einteilig ausgestaltet sind.

4. Vorrichtung (100) nach einem der vorhergehenden Ansprüche, **wobei** im Kanal (250) ein Filterelement (350) angeordnet ist.

5. Vorrichtung (100) nach Anspruch 4, **wobei** das Filterelement (350) im Kanal (250) proximal zum Rückschlagventil (400) angeordnet ist.

6. Vorrichtung (100) nach Anspruch 4 oder 5, **wobei** das Filterelement (350) eine mikroporöse Filterplatte ist.

7. Vorrichtung (100) nach Anspruch 6, **wobei** die mikroporöse Filterplatte Poren mit einem durchschnittlichen Porendurchmesser von kleiner 40 µm aufweist.

8. Vorrichtung (100) nach einem der vorhergehenden Ansprüche, **wobei** im Kanal (250) distal zum Dichtungselement (500) eine hohlzylinderförmige Hülse (360) angeordnet ist, um ein Austragen des Dichtungselements (500) aus dem distalen Kanalende (270) beim Applizieren des pharmazeutischen Fluids (650) zu unterbinden.

9. Vorrichtung (100) nach einem der vorhergehenden Ansprüche, wobei das proximale Kanalende (260) mehrkantförmig ausgebildet ist und das Verbindungselement (300) mit einem mehrkantförmigen Verbindungselementabschnitt (310) ausgestattet ist, um das Verbindungselement (300) mit dem proximalen Kanalende (210) reversibel fluidleitend durch Einstecken des Verbindungselementabschnitts (310) in das proximale Kanalende (260) zu verbinden.

10. Vorrichtung (100) nach Anspruch 9, **wobei** das mehrkantförmig ausgebildete proximale Kanalende (260) eine größere axiale Erstreckung aufweist als der mehrkantförmige Verbindungselementabschnitt (310), so dass nach einem Entfernen eines Teils des proximalen Kanalendes (260), insbesondere durch Absägen oder Abschneiden, das Verbindungselement (300) mit einem an der Vorrichtung (100) verbleibenden Teil des proximalen Kanalendes (260) fluidleitend durch Einstecken des Verbindungselementabschnitts (310) in das verbleibende proximale Kanalende (260) verbindbar ist.

11. Vorrichtung (100) nach einem der vorhergehenden Ansprüche, **wobei** der Körper (200) ein Metall und/oder ein Polymer umfasst.

## Claims

1. A device (100) for applying a pharmaceutical fluid (650) into a joint capsule, comprising a hollow cylindrical body (200) which surrounds a channel (250) extending through the body (200) from a proximal body end (210) to a distal body end (220) of the body (200), an outer surface of the body (200) at least partially comprising an external thread (230),
a connecting element (300) which is arranged at the proximal body end (210) and via which a proximal channel end (260) of the channel (250) can be reversibly connected to a reservoir (600) for the pharmaceutical fluid (650) in a fluid-conducting manner, and
a check valve (400) which is arranged in the channel (250) and is designed to be fluid-impermeable in the direction of the proximal channel end (260),
**characterized by**
a sealing element (500) which is arranged distally from the check valve (400) in the channel (250), closes a distal channel end (270) of the channel (250) in a fluid-conducting manner and comprises a gap (510) which can be reversibly opened in a fluid-conducting manner when pressure (660) is applied to the pharmaceutical fluid (650) from the direction of the proximal channel end (260), so that the pharmaceutical fluid (650) can be applied from the distal channel end (270) and the check valve (400) being a ball check valve comprising a ball (410) and a return element (420).

2. The device (100) according to claim 1, **wherein** the gap (510), when subjected to a pressure (660) greater than 5 N/cm², reversibly opens in a fluid-conducting manner.

3. The device (100) according to claim 1 or 2, **wherein** the return element (420) and the sealing element (500) are designed as a single unit.

4. The device (100) according to any of the preceding claims, **wherein** a filter element (350) is arranged in the channel (250).

5. The device (100) according to claim 4, **wherein** the filter element (350) is arranged in the channel (250) proximally to the check valve (400).

6. The device (100) according to claim 4 or 5, **wherein** the filter element (350) is a microporous filter plate.

7. The device (100) according to claim 6, **wherein** the microporous filter plate comprises pores with an average pore diameter of less than 40 µm.

8. The device (100) according to any of the preceding claims, **wherein** a hollow cylindrical sleeve (360) is arranged in the channel (250) distally from the sealing element (500) in order to prevent the sealing element (500) from being discharged from the distal channel end (270) when the pharmaceutical fluid (650) is applied.

9. The device (100) according to any of the preceding claims, **wherein** the proximal channel end (260) is polygonal and the connecting element (300) is equipped with a polygonal connecting element portion (310) in order to reversibly connect the connecting element (300) to the proximal channel end (210) in a fluid-conducting manner by inserting the connecting element portion (310) into the proximal channel end (260).

10. The device (100) according to claim 9, **wherein** the polygonal proximal channel end (260) has a greater axial extent than the polygonal connecting element portion (310), so that after removing part of the proximal channel end (260), in particular by sawing or cutting it off, the connecting element (300) can be connected in a fluid-conducting manner to a part of the proximal channel end (260) remaining on the device (100) by inserting the connecting element portion (310) into the remaining proximal channel end (260).

11. The device (100) according to any of the preceding claims, **wherein** the body (200) comprises a metal and/or a polymer.

## Revendications

1. Dispositif (100) pour l'application d'un fluide pharmaceutique (650) dans une capsule articulaire comprenant
un corps (200) en forme de cylindre creux qui entoure un canal (250), lequel canal s'étend à travers le corps (200) depuis une extrémité de corps proximale (210) jusqu'à une extrémité de corps distale (220) du corps (200),
dans lequel une surface extérieure du corps (200) présente au moins partiellement un filetage extérieur (230),
un élément de liaison (300) disposé à l'extrémité proximale de corps (210) et par l'intermédiaire duquel une extrémité proximale de canal (260) du canal (250) peut être reliée de manière réversible et d'une manière conductrice de fluide à un réservoir (600) pour le fluide pharmaceutique (650), et
un clapet antiretour (400) disposé dans le canal (250) et conçu de manière à être imperméable aux fluides en direction de l'extrémité proximale de canal (260),
**caractérisé par**
un élément d'étanchéité (500) disposé dans le canal (250) de manière distale par rapport au clapet antiretour (400), lequel élément d'étanchéité ferme une extrémité distale de canal (270) du canal (250) d'une manière conductrice de fluide et présente une fente (510) qui peut s'ouvrir de manière réversible et d'une manière conductrice de fluide lors d'une sollicitation par pression (660) sur le fluide pharmaceutique (650) depuis l'extrémité proximale de canal (260), de sorte que le fluide pharmaceutique (650) peut être appliqué à partir de l'extrémité distale de canal (270) et dans lequel le clapet antiretour (400) est un clapet antiretour à bille comprenant une bille (410) et un élément de rappel (420).

2. Dispositif (100) selon la revendication 1, **dans lequel** la fente (510) s'ouvre de manière réversible et d'une manière conductrice de fluide en cas de sollicitation par pression (660) supérieure à 5 N/cm².

3. Dispositif (100) selon la revendication 1 ou 2, **dans lequel** l'élément de rappel (420) et l'élément d'étanchéité (500) sont conçus d'une seule pièce.

4. Dispositif (100) selon l'une des revendications précédentes, **dans lequel** un élément filtrant (350) est disposé dans le canal (250).

5. Dispositif (100) selon la revendication 4, **dans lequel** l'élément filtrant (350) est disposé dans le canal (250) de manière proximale par rapport au clapet antiretour (400).

6. Dispositif (100) selon la revendication 4 ou 5, **dans lequel** l'élément filtrant (350) est une plaque filtrante microporeuse.

7. Dispositif (100) selon la revendication 6, **dans lequel** la plaque filtrante microporeuse présente des pores comportant un diamètre de pores moyen inférieur à 40 µm.

8. Dispositif (100) selon l'une des revendications précédentes, **dans lequel** un manchon (360) en forme de cylindre creux est disposé dans le canal (250) de manière distale par rapport à l'élément d'étanchéité (500) afin d'empêcher une sortie de l'élément d'étanchéité (500) hors de l'extrémité distale de canal (270) lors de l'application du fluide pharmaceutique (650).

9. Dispositif (100) selon l'une des revendications précédentes, **dans lequel** l'extrémité proximale de canal (260) est réalisée sous forme de polygone et l'élément de liaison (300) est équipé d'une section d'élément de liaison (310) sous forme de polygone afin de relier de manière réversible et d'une manière conductrice de fluide l'élément de liaison (300) à l'extrémité proximale de canal (210) par l'insertion de la section d'élément de liaison (310) dans l'extrémité proximale de canal (260).

10. Dispositif (100) selon la revendication 9, **dans lequel** l'extrémité proximale de canal (260) réalisée sous forme de polygone présente une extension axiale plus grande que la section d'élément de liaison (310) sous forme de polygone, de sorte qu'après un retrait d'une partie de l'extrémité proximale de canal (260), en particulier par sciage ou découpage, l'élément de liaison (300) peut être relié d'une manière conductrice de fluide à une partie de l'extrémité proximale de canal (260) restant sur le dispositif (100) par l'insertion de la section d'élément de liaison (310) dans l'extrémité proximale de canal (260) restante.

11. Dispositif (100) selon l'une des revendications précédentes, **dans lequel** le corps (200) comprend un métal et/ou un polymère.
